(19) Europäisches Patentamt
European Patent Office
Office européen des brevets

(11) EP 2 545 915 A1

(12) EUROPEAN PATENT APPLICATION

(43) Date of publication:
**16.01.2013 Bulletin 2013/03**

(21) Application number: **11305903.4**

(22) Date of filing: **11.07.2011**

(51) Int Cl.:
***A61K 31/357*** *(2006.01)* ***A61K 31/485*** *(2006.01)*
***A61K 45/06*** *(2006.01)* ***A61P 25/04*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **SANOFI**
**75008 Paris (FR)**

(72) Inventor: **The designation of the inventor has not yet been filed**

(74) Representative: **Tinel, Marie-Line et al**
**Sanofi**
**Département Brevets**
**174, avenue de France**
**75013 Paris (FR)**

(54) **2-amino-2-oxoethyl trans-3-[5-(6-methoxy-naphtalen-1-yl)-1,3-dioxan-2-yl]propyl-carbamate for use in the treatment of persistent cancer pain**

(57) The present invention relates to 2-amino-2-oxoethyl trans-3-[5-(6-methoxy-naphtalen-1-yl)-1,3-dioxan-2-yl]propyl-carbamate (hereinafter referred to as compound A) for use in the treatment of persistent cancer pain, alone or as adjunctive treatment

EP 2 545 915 A1

## Description

The present invention relates to 2-amino-2-oxoethyl trans-3-[5-(6-methoxy-naphtalen-1-yl)-1,3-dioxan-2-yl]propyl-carbamate (hereinafter referred to as compound A) for use in the treatment of persistent cancer pain, alone or as adjunctive treatment.

The compound 2-amino-2-oxoethyl trans-3-[5-(6-methoxy-naphtalen-1-yl)-1,3-dioxan-2-yl]propyl-carbamate and a preparative method are described in the patent application W02004/0204303. Compound A is a FAAH inhibitor.

The compounds described in this document can be used in the prevention and treatment of any pathological condition in which endogenous cannabinoids and/or any other substrate metabolized by the FAAH enzyme are involved. Among other pathological conditions, pain is mentioned, notably acute or chronic pain of the neurogenic type: migraine, neuropathic pain including forms associated with the herpes virus and with diabetes; acute or chronic pain associated with inflammatory diseases, acute or chronic peripheral pain.

Other pain should also be considered. There is an increase of medical need such as the treatment of persistent cancer pain.

Cancer pain is complex and involves mixed types of pain from multiple causes. Cancer patients usually experience more than one pain type, making accurate diagnosis and selection of treatment difficult.

The present invention relates to 2-amino-2-oxoethyl trans-3-[5-(6-methoxy-naphtalen-1-yl)-1,3-dioxan-2-yl]propyl-carbamate for use in the treatment of persistent cancer pain.

In some cases, patients with persistent cancer pain have a background therapy which consists of opioids ± non-opioids ± adjuvants. Therefore it is necessary for adjunctive treatment not to interfere with the background therapy for pain. According to in vitro results, compound A has no significant drug interaction with morphine that is essential to allow its use as adjunctive treatment for persistent cancer pain.

The invention is related to compound A for use in the treatment of persistent cancer pain. It is also to considered that the invention is related to compound A for use in the treatment of persistent pain in cancer patients.

In another aspect, the invention is related to compound A for use in the treatment of persistent cancer pain wherein pain generator is primarily due to underlying cancer.

In another aspect, the invention is related to compound A for use in the treatment of persistent cancer pain wherein pain generator is primarily due to cancer treatment.

In another aspect, the invention is related to compound A for use in the treatment of persistent cancer pain wherein pain generator is classified as primarly nociceptive.

In another aspect, the invention is related to compound A for use in the treatment of persistent cancer pain wherein pain generator is classified as primarly neuropathic.

In another aspect, the invention is related to compound A for use in the treatment of persistent cancer pain wherein the persistent cancer pain is bone cancer.

In another aspect, the invention is related to compound A for use in the treatment of persistent cancer pain wherein persistent cancer pain is metastatic cancer.

In another aspect, the invention is related to compound A for use in the treatment of persistent cancer pain wherein persistent cancer pain is metastatic bone cancer.

In another aspect, the invention is related to compound A for use in a therapeutic treatment to reduce pain intensity.

In another aspect, the invention is related to compound A for use in a therapeutic treatment to reduce breakthrough pain frequency.

In other aspects, the invention is related to compound A for each uses as specified before in the treatment of persistent cancer pain as adjunctive treatment.

In another aspect, the invention is related to compound A for use in a therapeutic treatment to reduce utilization of background therapy.

In another aspect, the invention is related to use of compound A and morphine in the treatment of persistent cancer pain. In another aspect, the invention is related to use of compound A and morphine derivative in the treatment of persistent cancer pain.

In another aspect, the invention is related to use of compound A and opioids in the treatment of persistent cancer pain.

The use of compound A and background therapy can be simultaneously, separately or sequentially.

In another aspect, the invention is related to compound A for use as adjunctive treatment in the treatment of persistent cancer pain, wherein compound A is administered to

- patient with moderate or severe, persistent cancer pain who are receiving the World Health Organization (WHO) Step 2 or 3 cancer pain treatment.
- pain generator is primarily due to underlying cancer or cancer treatment.

- pain generator is classified as either primarily nociceptive or primarily neuropathic.
- pain severity is moderate or severe as defined by the Numeric Rating Scale during the screening phase

In another aspect, the invention is related to compound A for use as adjunctive treatment in the treatment of persistent cancer pain, wherein compound A is administered to patient with moderate or severe, persistent cancer pain who are receiving the World Health Organization (WHO) Step 2 or 3 cancer pain treatment.
In another aspect, the invention is related to compound A for use as adjunctive treatment in the treatment of persistent cancer pain, wherein pain generator is primarily due to underlying cancer.
In another aspect, the invention is related to compound A for use as adjunctive treatment in the treatment of persistent cancer pain, wherein pain generator is primarily due to cancer treatment.
In another aspect, the invention is related to compound A for use as adjunctive treatment in the treatment of persistent cancer pain, wherein pain generator is classified as either primarily nociceptive or primarily neuropathic.
In another aspect, the invention is related to compound A for use as adjunctive treatment in the treatment of persistent cancer pain, wherein pain generator is classified as primarily nociceptive.
In another aspect, the invention is related to compound A for use as adjunctive treatment in the treatment of persistent cancer pain, wherein pain generator is classified as primarily neuropathic.
In another aspect, the invention is related to compound A for use as adjunctive treatment in the treatment of persistent cancer pain, wherein pain severity is moderate or severe as defined by the Numeric Rating Scale during the screening phase.
In another aspect, the invention is related to compound A for use as adjunctive treatment in the treatment of persistent cancer pain, wherein pain severity is moderate as defined by the Numeric Rating Scale during the screening phase.
In another aspect, the invention is related to compound A for use as adjunctive treatment in the treatment of persistent cancer pain, wherein pain severity is severe as defined by the Numeric Rating Scale during the screening phase.
In a further aspect of the invention, it is to be understood that the patients having the following caracteristics are not included in the scope of the invention and/or the claimed use and/or technical effect:

- pain is not stable at study visit 1 (screening visit) and visit 2 (randomization visit)
- use of prohibited adjuvant pain treatment in the week prior to visit 1 or plan to use these medications during the study
- current use of medication containing tetrahydrocannabinol (THC)
- chemotherapy within 4 weeks of visit 1 or chemotherapy planned during the study
- radiotherapy within 4 weeks of visit 1 or radiotherapy planned during the study (hemostatic palliative radiotherapy is permitted)
- cancer related surgery within 4 weeks of visit 1 or cancer-related surgery planned during the study

In accordance with what is explained just before, in another aspect, the invention is related to compound A for use as adjunctive treatment in the treatment of persistent cancer pain, wherein:

- pain is stable at study visit 1 (screening visit) and visit 2 (randomization visit), and/or
- use of adjuvant pain treatment in the week prior to visit 1 or plan to use these medications during the study is prohibited, and/or
- the patient does not currently use of medication containing tetrahydrocannabinol (THC), and/or
- the patient does not have chemotherapy within 4 weeks of visit 1 or chemotherapy planned during the study, and/or
- the patient does not have radiotherapy within 4 weeks of visit 1 or radiotherapy planned during the study (hemostatic palliative radiotherapy is permitted), and/or
- the patient does not have cancer related surgery within 4 weeks of visit 1 or cancer-related surgery planned during the study.

In another aspect, the invention is related to compound A for use as adjunctive treatment in the treatment of persistent cancer pain, measured by the change of pain severity from baseline using the Numeric Rating Scale.
More particularly, the invention is related to compound A for use as adjunctive treatment in the treatment of persistent cancer pain, measured by change from baseline (baseline = average pain intensity between visit 1 and visit 2) to end of treatment (end of treatment = average pain intensity during week 4) in cancer pain severity assessed by the Numeric Rating Scale.
More particularly, the invention is related to compound A for use as adjunctive treatment in the treatment of persistent cancer pain measured by Brief Pain Inventory Short-Form (BPI-SF).
In another aspect, the invention is related to compound A for use as adjunctive treatment in the treatment of

persistent cancer pain, measured by reduction of pain intensity.

In another aspect, the invention is related to compound A for use as adjunctive treatment in the treatment of persistent cancer pain, measured by responder rate.

In another aspect, the invention is related to compound A for use as adjunctive treatment in the treatment of persistent cancer pain, measured by reduction of breakthrough pain frequency.

In another aspect, the invention is related to compound A for use as adjunctive treatment in the treatment of persistent cancer pain, measured by reduction of background therapy utilization.

In another aspect, the invention is related to compound A for use as adjunctive treatment in the treatment of persistent cancer pain and to reduce background therapy utilization.

In another aspect, the invention is related to compound A for use as adjunctive treatment in the treatment of persistent cancer pain, measured by improvement of mood.

In another aspect, the invention is related to compound A for use as adjunctive treatment in the treatment of persistent cancer pain, and to improve mood.

In another aspect, the invention is related to compound A for use as adjunctive treatment in the treatment of persistent cancer pain, measured by improvement of patient satisfaction of pain relief.

In another aspect, the invention is related to compound A for use as adjunctive treatment in the treatment of persistent cancer pain, and to improve patient satisfaction of pain relief.

In another aspect, the invention is related to compound A for use as adjunctive treatment in the treatment of persistent cancer pain, measured by reduction of constipation.

In another aspect, the invention is related to compound A for use as adjunctive treatment in the treatment of persistent cancer pain, and to reduce constipation.

In another aspect, the invention is related to compound A for use as adjunctive treatment in the treatment of persistent cancer pain, measured by improvement in Nausea visual analog scale.

In another aspect, the invention is related to compound A for use as adjunctive treatment in the treatment of persistent cancer pain, and to improve Nausea visual analog scale.

In another aspect, the invention is related to compound A for use as adjunctive treatment in the treatment of persistent cancer pain, measured by heathcare utilization.

In another aspect, the invention is related to compound A for use as adjunctive treatment in the treatment of persistent cancer pain, measured by improvement of quality of life.

In another aspect, the invention is related to compound A for use as adjunctive treatment in the treatment of persistent cancer pain, measured by one or several "indicator" selected from the following list : change of pain severity from baseline using the Numeric Rating Scale, reduction of pain intensity, responder rate, breakthrough pain frequency, background therapy utilization, mood, patient satisfaction of pain relief, constipation, heathcare utilization, quality of life.

In another aspect, the invention is related to compound A for use in the treatment of persistent cancer pain wherein compound A is administered 200 mg once daily. In another aspect, the invention is related to compound A for use in the treatment of persistent cancer pain wherein compound A is administered 200 mg once daily with food. In another aspect, the invention is related to compound A for use in the treatment of persistent cancer pain wherein compound A is administered 200 mg once daily in the morning with food.

There may be specific cases where higher or lower dosages are appropriate; such dosages do not depart from the context of the invention. According to the usual practice, the dosage appropriate for each patient is determined by the physician according to the method of administration, the weight, the pathology, the body surface, the cardiac output and the response of said patient.

One or several of each aspects of the invention cited before can be combined independently with one or several other aspects of the invention also cited before to provide another aspect of the invention.

Is it to be understood that "compound A for use in the treatment of" has an equivalent meaning to "Use of compound A for the treatment of", "Use of compound A for the manufacture of a medicament useful for the treatment of".

In another aspect, the invention is related to a pharmaceutical composition comprising 2-amino-2-oxoethyl trans-3-[5-(6-methoxy-naphtalen-1-yl)-1,3-dioxan-2-yl]propyl-carbamate (compound A) as active ingredient and pharmaceutically acceptable excipients for use in the treatment of persistent cancer pain. More particularly, the invention is also related to this pharmaceutical composition any one of the uses as described before.

In another aspect, the invention is related to a medicament comprising 2-amino-2-oxoethyl trans-3-[5-(6-methoxy-naphtalen-1-yl)-1,3-dioxan-2-yl]propyl-carbamate as active ingredient for use in the treatment of persistent cancer pain. More particularly, the invention is also related to a medicament for any one of the uses as described before.

In another aspect, the invention is related to compound A for use in the treatment of persistent cancer pain wherein compound A is administered as a tablet having the following composition:

| Ingredients | [mg] |
|---|---|
| Compound A | 200 |
| Lactose monohydrate | 106 |
| Maize starch | 53 |
| Hypromellose | 16,5 |
| Crospovidone | 20,5 |
| Magnesium stearate | 4 |
| total weight [mg] | 400 |

According to another of its aspects, the present invention also relates to a method for treating cancer pain as indicated above, which comprises the administration, to a patient, of an effective dose of compound A or a pharmaceutically acceptable salt thereof.

Other subject matters of the invention are the methods of treatment for the different aspects of the invention described before and the methods of treatment comprising the uses of compound A as described before.

In another aspect, the invention is related to a method of reducing pain intensity by administering compound A to a patient having persistent cancer pain. In another aspect, the invention is related to a method of reducing breakthrough pain frequency by administering compound A to a patient having persistent cancer pain. In another aspect, the invention is related to a method of reducing utilization of background therapy by administering compound A to a patient having persistent cancer pain.

In another aspect, the invention is related to an article of manufacture comprising a packaging material; compound A; and a label or package insert contained within the packaging material indicating that patients receiving the treatment with the above mentioned pharmaceutical composition can be treated for persistent cancer can be treated for persistent cancer pain.

In another aspect, the invention is related to a method of treating a human subject having persistent cancer pain comprising administering a pharmaceutical composition comprising compound A to the subject.

In another aspect, the invention is related to a method of preventing pain associated with cancer comprising administering a pharmaceutical composition comprising compound A to a patient having cancer, such that pain is prevented.

<u>**Definitions**</u>

**Compound A** is 2-amino-2-oxoethyl trans-3-[5-(6-methoxy-naphtalen-1-yl)-1,3-dioxan-2-yl]propyl-carbamate having the following chemical structure:

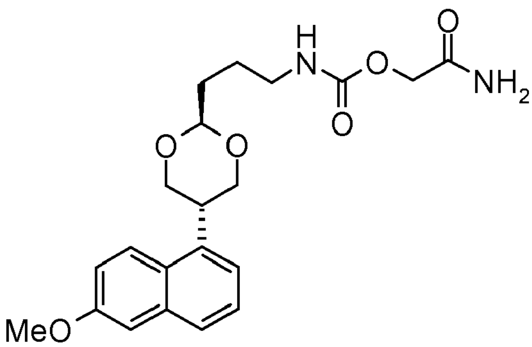

**Cancer pain** is a chronic moderate or severe pain defined as complex pain modulated by one or several pathophysiological mechanisms (visceral, somatic, neuropathic). The two most common causes of cancer pain are the cancer itself and the treatments used to treat cancer.

- The cancer itself. When cancer causes pain, some probable causes include the pressure of a tumor on one of the body's organs or on bone or nerves.
- Cancer treatments. Some examples of treatment-related pain include:

- Chemotherapy can cause numerous side effects, depending in the medication being used. Some of the more common side effects that cause pain include mouth sores (mucositis), peripheral neuropathy (numb and sometimes painful sensations in the feet, legs, fingers, hands and arms), constipation, diarrhea, nausea, vomiting and abdominal cramps. Some people also experience bone and joint pain from chemotherapy medications and from some medications used to offset the impact of the chemotherapy on blood counts and on the risk of infection.
- Radiotherapy which may cause tissue damage leading in some cases to nociceptive pain.
- Surgical treatments will, in some instances produce pain after they are completed.
- Procedures related to cancer pain, such as biopsies, blood draws, lumbar punctures, laser treatments, etc. can cause pain.

**Persistent cancer pain** is defined as chronic pain for which relief is not obtainable with existing pain treatments.
Three broad categories of cancer pain may be considered alone or concomitantly:
**Nociceptive cancer pain** is pain caused by tissue damage created by pressure, infiltration or destruction by an identifiable visceral or somatic lesion.

■ **Visceral pain** is constant, dull, aching, poorly localized pain that has a gradual onset often felt at a distance from the origin.
■ **Somatic pain** is constant gnawing or aching, usually well localized, worse on movement or weight-bearing if in pelvis, hips, femur, joints or spine.
■ Within nociceptive pain, bone cancer represents a particular subcategory with mixed syndromes of nociceptive and neuropathic pain types. However it is acknowledged that the skilled man in the art, such as an investigator, physician or doctor having skills in palliative care and management of pain is able to differentiate whether pain is

primarily from nociceptive or neuropathic origin.
**Neuropathic cancer pain** is pain caused by pressure, invasion or destruction of peripheral or central nervous tissues, which leads to complex and abnormal spinal cord or thalamic neural processes that produce sustained pain.
The abnormal sensation or hypersensitivity in the affected area may be characterized by:

- Paraesthesias: skin crawling sensation or tingling
- Spontaneous ongoing pain, burning, shooting, electric shock-like sensations
- Accompanying somatosensory disturbance in the painful territory

  o Numbness
  o Allodynia: provocation of pain by normally innocuous stimuli such as light touch
  o Hyperalgesia: exaggerated response to painful stimuli
  o Summation: progressive worsening of pain evoked by slow repetitive stimulation with mildly noxious stimuli

**Neurogenic pain** is pain caused or affected by nerve or nervous tissue damage.
**Moderate or severe** cancer pain is defined according to a pain severity scale such as NRS and/or BPI-SF.
**Breakthrough pain** is an acute flare of pain that 'breaks through' the pain analgesia.
**Pain generator** is defined as the source of pain.
**Adjunctive treatment is a background therapy** for the treatment of pain and is defined as opioids $\pm$ non-opioids $\pm$ adjuvants $\pm$ rescue therapy for breakthrough pain.
**Opioids** refer to, it can be mentioned: morphine, fentanyl, codeine, hydrocodeine, oxycodone, acetaminophen/paracetamol, hydrocodone/tramadol, lidocaine, methadone, meperidine, tramadol. This list is not limitative.
**Non-opiods** refer to NSAIDs, Paracetamol, COX 2 inhibitor, antiepileptics, anti-depressant, benzodiazepine, tranquillisers, biphosponates.
**NSAIDs** refer to acetylsalicylic acid (aspirin), diclofenac, ibuprofen, indomethacin, sulindac, ketoprofen, loxoprofen, naproxen, oxaprozin.
**Cox2 inhibitors** refer to celecoxib, etoricoxib, valdecoxib, parecoxib.
**Anti-epileptics** refer to Calcium channel $\alpha 2$-$\delta$ ligands such as Gabapentin and pregabalin.
**Antidepressants** refer to Tricyclic antidepressants, Selective serotonin reuptake inhibitors (SSNRIs) such as duloxetine and venlafaxine.
The **NRS** (numeric rating scale) is an 11-category (from 0 [no pain] to 10 [worst possible pain]), descriptive-anchor scale that is one of the most frequently employed and accepted scales for pain evaluation. NRS allows a discontinuous 0 to 10 data collection between the same boundaries. While the NRS and the Visual Analogue Scale (VAS) demonstrate similar sensitivity of rating in pain intensity, the choice of the NRS is based on the fact that this scale is validated in cancer pain population.

The **BPI-SF** (Brief Pain Inventory Short-Form) was developed originally for use with cancer patients and it has been adopted widely for clinical pain assessment, epidemiological studies, and in studies of effectiveness of pain treatment. Nine questions measure the intensity of pain, interference of pain, pain relief, pain quality, and patient perception of the cause of pain.

The **HADS** (Hospital Anxiety and Depression Scale) was designed to screen for the presence of a mood disorder in medically ill patients. This self-reported scale contains 14 items rated on 4-point Likert scales. Two subscales assess depression (7 items) and anxiety (7 items). Each 7-item subscale yields a score of 0 to 21 that is interpreted with the following cut points: 0-7, normal; 8-10, mild mood disturbance; 11-14, moderate mood disturbance; and 12-21, severe mood disturbance.

The BFI (Bowel Function Index) is a 3-item questionnaire to measure constipation from the patient's perspective. The time frame for the questions is "during the last 7 days". The BFI is administered by a clinician. The answer for each of the 3 items is rated on a scale from 0 to 100, where 0 = easy or no difficulty and 100 = severe difficulty.

**Healthcare utilization** measures patients'overall healthcare needs including unscheduled hospitalizations, emergency department visits, healthcare provider office visits, and sick leave days

**Patient satisfaction** of pain relief is a 5-point Likert scale that measures patient satisfaction with treatment. The five categorical responses are: extremely unsatisfied, unsatisfied, neutral, satisfied, extremely satisfied.

The **EORTC QLQ-C30** (European Organization For Research And Treatment Of Cancer Quality Of Life Questionnaire-C3 Version 3) was designed evaluate the quality of life (QoL) of cancer patients participating in international clinical trials. The questionnaire contains a total of 30 items, of which 28 items are rated on 4-point Likert-type scale and 2 items are rated on 7-point Likert scale. These include 5 functional scales, 3 symptom scales, a global health status/QoL scale, and 6 single items. A high scale score represents a higher response level. Thus a high score for a functional scale represents a high/healthy level of functioning, a high score for the global health status/QoL represents a high QoL, but a high score for a symptom scale/item represents a high level of symptomatology/problems.

WHO: World Health Organization
THC: tetrahydrocannabinol
Conc.: Concentration
CV: Coefficient of variance
ESI: Electrospray ionization
eq: Equivalent
g: Gram(s)
x g: Times gravity
HLM Human Liver Microsomes
HPLC: High-performance liquid chromatography
hr: Hour(s)
$K_m$ : Michaelis-Menten constant
LC-MS/MS: Liquid chromatography-tandem mass spectrometry
MBL: Medpace Bioanalytical Laboratory
mg: Milligram(s)
min (m): Minute(s)
mL: Milliliter(s)
mm: Millimeter(s)
mM: Millimolar(s)
MRM: Multiple reaction monitoring
μg: Microgram(s)
μL: Microliter(s)
μm: Micrometer(s)
μM: Micromolar(s)
$N_2$: Nitrogen
Rt: Retention time
SD: Standard deviation
STD: Standard
UDPGA: Uridine 5'-diphosphoglucuronic acid
UGT: UDP-glucuronosyl transferase
V: Volt(s)
$V_{max}$ : Maximum enzyme velocity

The present invention is illustrated by the data hereinafter.

**Example 1: Drug-Drug Interactions study with Morphine**

The inhibition of UDP-glucuronosyl tranferase mediated metabolism of morphine glucuronidation by Compound A was investigated in human liver microsomes, by monitoring the formation of two prominent metabolites (morphine-3-glucuronide and morphine-6-glucuronide). Compound A showed no inhibition of morphine metabolism under the conditions employed in this *in vitro* study.

**1. Materials**

**1.1 Reference Standards**

The test article is compound A

**1.2 Test Systems** Human liver microsomes (pool of 10, product number: X008061) were obtained from Celsis.

**1.3 Solvents, Reagents, and Chemicals**

Water ($H_2O$): HPLC Grade, Fisher, or equivalent
Methanol ($CH_3OH$): HPLC Grade, Fisher, or equivalent
Acetonitrile (ACN): HPLC Grade, Fisher, or equivalent
Ammonium Acetate: HPLC Grade, Fisher, or equivalent
Formic acid: 96% ACS, Sigma-Aldrich, or equivalent
$K_2HPO_4$: 98% ACS, Sigma-Aldrich, or equivalent
$KH_2PO_4$: 99% ACS, Sigma-Aldrich, or equivalent
$MgCl_2$: 98% ACS, Sigma-Aldrich, or equivalent
DMSO: 99.9% ACS, Sigma-Aldrich, or equivalent

Morphine (1.0 mg/mL), M3G (100 μg/mL), and M6G (100 μg/mL) for analysis were purchased from Cerilliant. Morphine sulfate salt pentahrdrate, acetaminophen, uridine 5'-diphosphoglucuronic acid triammonium salt (UDPGA, 99.3%), D-saccharic acid-1,4-lactone monohydrate, and alamethicin (from *Trichoderma viride*) were acquired from Sigma-Aldrich.

**1.4 Equipment**

The following equipment and supplies can be used. Equipment and supplies, which are capable of delivering equivalent performance from alternative manufacturers, may be substituted. The miscellaneous glassware can be used for preparation of standard, QC solutions and samples.

Balances: Mettler Toledo, MX-5, XS205DU, PL202-S or equivalent

Centrifuges: Eppendorf 5810, 5810R or equivalent

Vortexers: VWR Scientific Multi-Tube Vortexer, Thermo MaxMix II Vortex Mixer, Fisher Genie 2, or equivalent

Evaporator: Caliper TurboVap® LV Evaporator, or equivalent

Disposable Pipettes: 1 mL in 1/100, 5 mL in 1/10, and 10 mL in 1/10, VWR, or equivalent Micro-Pipettes: 1-5, 25, 50, and 100 μL, Eppendorf, or equivalent

Automatic Pipettes: 10-100 μL, 20-200 μL, and 100-1,000 μL, Eppendorf, or equivalent Sonicator: Branson 5510, 1510, or equivalent

Autosampler Vials: 1.5-mL Amber Glass Vials (SUN-SRi, Cat. No. 200 252) or equivalent

pH Meter: Mettler-Toledo

Water Baths: Fisher Scientific.

Centrifuge Tubes with Screw Caps: 2-mL (VWR, Cat. No. 20170-211) or equivalent

96-well plate: True Taper 2-mL 96-well plate (Analytical Sales & Products, Inc.) or equivalent Miscellaneous bottles, vials, tubes and various glassware.

**1.5 LC-MS/MS Conditions**

A reverse phase HPLC column was used for the separation of the analyte. The analyte was eluted using a gradient of mobile phase A (HPLC water with 5 mM ammonium acetate and 0.1 % formic acid) and mobile phase B ($CH_3OH$/water (90/10) with 5 mM ammonium acetate and 0.1 % formic acid) from 2% to 40% mobile phase B. Morphine, M3G, M6G and the internal standard were detected using the following conditions: Mass Spectrometer: MDS Sciex API

4000 LC-MS/MS System, or equivalent ; Ionization Mode: Turbo IonSpray®, Positive Ion Detection ; Scan Mode: Multiple Reaction Monitoring (MRM) ; Ion Spray Voltage (IS): 5,500 V ; Temperature (TEM): 500 °C ; Curtain Gas ($N_2$) (CUR): 20 ; Collision Gas (CAD): 6 ; Gas 1: 60 ; Gas 2: 60 ; Declustering Potential (DP): 30 ; Entrance Potential (EP): 10; Potential (CXP): 12.

Precursor Ion and Product Ion

| Compound | Precursor → Product Transition (m/z) | Collision Energy (CE) | Dwell Time (msec) |
|---|---|---|---|
| Morphine | 286.1 →201.1 | 50 V | 200 |
| Morphine-3-glucuronide (M3G) | 462.0 →286.3 | 36 V | 200 |
| Morphine-6-glucuronide (M6G) | 462.0 →286.3 | 36 V | 200 |
| Acetaminophen (IS) | 152.2 →110.0 | 20 V | 100 |

**2. Methods**

**2.1 Preparation of HLM Incubation**

**2.1.1 Preparation of Solutions**

Stock solutions of morphine were prepared in suitable organic solvents and stored at -20 °C prior to use. Working stock solutions were freshly prepared before each incubation in potassium phosphate buffer (0.1 M, pH 7.4) and kept on ice prior to use.

Various concentration of Compound A was prepared in DMSO and stored at -20 °C until use.

Selective inhibitor, ketoconazole, was prepared in DMSO and stored at -20 °C until use. Acetaminophen was used as the IS for the determination of morphine, M3G, and M6G. Stock and working solutions of acetaminophen were prepared in methanol and potassium phosphate buffer (0.1 M, pH 7.4). Solutions were stored at -20 °C prior to use. Acetonitrile was used as quench solution in the study.

**2.1.2 General HLM Incubation Procedure**

Incubations were conducted in 2-mL polypropylene centrifuge tubes. Incubation mixtures containing HLM suspension and alamethicin (25 μg/mL) in 0.1 M potassium phosphate buffer (pH 7.4), were placed on wet ice for 5 min. $MgCl_2$, morphine, and saccharolactone were added and pre-incubated at 37 °C for 5 min in a water bath. Reactions were initiated by the addition of 50 mM UDPGA spiking solution. The final incubation mixture contained 0.1 mg (0.5 mg/mL) HLM, 2 mM $MgCl_2$ 5 mM UDPGA, and morphine over a concentration range of 0.5 - 7.5 mM. Samples were incubated aerobically in a water bath at 37 °C for the required amount of time and reactions terminated by the addition of 400 μL of the appropriate quench solution. The 2-mL centrifuge tubes were vortex mixed and stored in a -20 °C freezer for ca. 20 min prior to centrifugation at 13.3 × g, 4 °C for 10 min. Supernatants were analyzed by LC-MS/MS for morphine metabolites formation.

**2.2 Determination of Kinetic Parameters for Morphine Metabolism in HLM**

The kinetic parameters $K_m$ and $V_{max}$ for two prominent metabolites of morphine in HLM were determined. Prior to the determination of the kinetic parameters, the metabolism of morphine with respect to incubation time and protein concentration was evaluated. At each timepoint, the incubation mixture was taken and quenched by the addition of 2 volumes of ice-cold Quench Solution. The samples were centrifuged and the resultant supernatants were analyzed for the formation of metabolites M3G and M6G, using LC-MS/MS.

**2.2.1 Morphine Metabolism in HLM vs. Time**

Morphine metabolism as a function of time was determined by incubating morphine with HLM (0.5 mg/mL) at 2.0 mM for 0, 15, 30, 45, and 60 min.

### 2.2.2 Morphine Metabolism in HLM vs. Protein Concentration

Morphine metabolism as a function of protein concentration was determined by incubating morphine at 2.0 mM with 0, 0.25, 0.5 and 1.0 mg/mL HLM for 30 min.

### 2.2.3 Determination of $K_m$ and $V_{max}$

Determination of the kinetic parameters $K_m$ and $V_{max}$ values for two prominent metabolites of morphine in HLM was performed under conditions shown to be linear with respect to time and protein concentration. Kinetic parameters were determined by incubating morphine at 0.1, 0.25, 0.5, 1.0, 2.0, 2.5, 5.0, and 7.5 mM with HLM at 0.5 mg/mL for 30 min.

### 2.3 UDP-Glucuronosyl Transferase-Dependent Activities

UGT-dependent activities were determined by monitoring the formation of enzyme specific metabolites from marker substrates. For the reaction, formation of the metabolite of morphine in the presence (0.5, 1, 5, 10, 25, 50, 100, and 250 μM) and absence (positive control) of Compound A were measured. Incubation with selective inhibitors (positive control) was performed at a concentration above their established IC50 for the confirmation of enzyme activity and inhibitory effects.

### 2.4 Evaluation of Morphine Metabolism in HLM in the Presence of Compound A

Incubations were performed in 2-mL centrifuge tubes. Incubation mixtures containing HLM suspension and alamethicin (25 μg/mL) in 0.1 M potassium phosphate buffer (pH 7.4), were placed on wet ice for 5 min. $MgCl_2$, morphine, Compound A or selective inhibitor (added from 100X working solution) and saccharolactone were added and pre-incubated at 37 °C for 5 min in a water bath. Reactions were initiated by the addition of UDPGA. The final incubation mixture contained 0.5 mg/mL HLM, 2 mM $MgCl_2$, 5 mM UDPGA, various concentration of Compound A or a selective inhibitor. Samples were incubated aerobically in a water bath at 37 °C for 30 min. The incubation systems were terminated by the addition of a 400-μL quenching solution. The 2-mL centrifuge tubes were vortex mixed and stored in a -20 °C freezer for approximately 20 min prior to centrifugation at 13.3 × g, 4 °C for 10 min. Supernatants were analyzed by LC-MS/MS.

## 3. Determination of metabolites

### 3.1 Standard and QC Samples

The concentrations of the metabolites from the marker substrate reactions were determined by LC-MS/MS. Appropriate concentrations of calibration standards (eight) and QCs (3 levels) of individual metabolites were prepared in HLM extract.

### 3.2 Quench Solution and IS Solution Preparation

Acetonitrile was used as quench solution and kept on ice prior to use. After the supernatant was transferred into 96-well plate, 10 μL of IS-C was added into each sample.

### 3.3 Data Processing

The software provided with the LC-MS/MS system (Analyst™ software, version 1.5) was used to integrate the area under the mass ion peaks of the analytes and internal standards.

The data were subsequently imported into Microsoft® Excel spreadsheets, version 2003, and the report was prepared using Microsoft® Word for Windows version XP software. The entries were verified against the raw data. Due to rounding, data may vary by ±0.01% for concentration or recovery.

### 3.4 Accuracy and Precision

Accuracy, defined as the closeness of agreement between the test result and the nominal value, was expressed as the mean recovery. Precision, defined as the variability of measurements within replicates, was represented by the percent coefficient of variation.

### 3.5 Acceptance Criteria

The criteria for an acceptable batch are: For linearity, the calibration curve should have yielded a correlation coefficient (r) value of 0.990 or greater, and at least four of the back-calculated concentrations of individual standards should have been within ±15% of the nominal values, except for the lowest concentration standard where within ±20% of the nominal values was allowed.

For accuracy, the mean values of the intra-day samples at each QC concentration should be within 85-115% of the nominal value and for precision, CV of the intra-day QC samples at each QC concentration should be within 15%.

### 3.6 Calculations

### 3.6.1 LC/MS Quantitation

The software Analyst 1.5 provided with the LC-MS/MS system by Applied Biosystems/MDS Sciex will be used to integrate the area of the mass ion peaks for the analytes and the internal standard.

The peak area ratio (analyte/IS) vs. nominal concentrations of the calibration standards will be used to determine the concentration of samples and QCs with a weighted linear regression.

Calibration Curve

Calibration standards will be prepared at eight concentration levels in blank incubation matrix. The calibration curve will be determined from peak area ratio vs. concentration using a weighted linear regression: y = mx + b where x = Analyte calibration standard concentration ; y = Peak area ratio (i.e., analyte/IS) ; b = Intercept ; m = Slope.

Calculation of Analyte Concentration

Once the values of *m* and *b* are established and the calibration curve meets acceptance criteria, the *m* and *b* values will be used to calculate the concentrations of analyte in each sample by the following equation:

$$x = \frac{y - b}{m}$$

where x = Concentration of analyte ; y = Peak area ratio (analyte/IS) ; b = Intercept ; m = Slope.

Calculation of Mean Value

$$\text{MeanValue}(\overline{X}) = \frac{\sum_{i=1}^{n} \text{individual data (X)}}{\text{number of data points (n)}}$$

Calculation of Standard Deviation

$$\text{Standard Deviation (SD)} = \sqrt{\frac{\sum_{i=1}^{n} [\text{individual data (X)} - \text{Mean Value}(\overline{X})]^2}{n-1}}$$

### 3.6.2 Michaelis-Menten Parameter Determination

The Michaelis-Menten equation describes the kinetics of an enzyme reaction under pseudo first order conditions - the initial reaction velocity ($V_o$) is linear with respect to reaction time and enzyme concentration. Under steady state conditions with respect to the formation of any intermediates, the formation of product P per unit time is expressed as:

$$V = \frac{dP}{dt} = \frac{V_{max}[S]}{K_m + [S]},$$

where $V_{max}$ is the maximum enzyme velocity, attained when the enzyme sites are saturated with substrate [S]. $K_m$ is the Michaelis constant such that when

$$K_m = [S], \text{ then } V = \frac{V_{max}}{2}$$

The apparent values for $K_m$ and $V_{max}$ were determined by non-linear regression analysis of the Michaelis-Menten equation using GraphPad Prism version 5.01 for Windows (GraphPad Software, San Diego California USA).

**3.6.3 Percent of Control and IC50**

Percent of Control = Cmet ÷ Ccontrol × 100%

Cmet: Concentration of the metabolite in the incubations in the presence of Compound A or selective inhibitor

Ccontrol: Concentration of the enzyme-specific metabolite in the incubations in the absence of Compound A and selective inhibitor

The $IC_{50}$ for Compound A was estimated by non-linear regression analysis of individual sets of percent inhibition values using GraphPad Prism 5.

**4. Results and discussion**

**4.1 Protein Concentration Evaluation**

The formation of M3G and M6G appeared to be linear up to a protein concentration of 1.0 mg/mL and up to 60 min. The microsomal protein concentration 0.5 mg/mL was used for all subsequent incubations.

**4.2 Time Course Evaluation**

The formation of M3G and M6G appeared to be linear up to a 30 min incubation. A 30-min incubation was used for all subsequent incubations.

**4.3 Determination of $K_m$ and $V_{max}$ for Morphine**

Morphine was incubated at eight concentrations 0.1, 0.25, 0.5, 1.0, 2.0, 2.5, 5.0, and 7.5 mM with HLM at 0.5 mg/mL for 30 min. The metabolic parameter $K_m$ was estimated using the Michaelis-Menten equations. $K_m$ values were 2.59 mM and 2.11 mM for M3G and M6G, respectively.

**4.4 Compound A Inhibition of Morphine Metabolism in Human Liver Microsomes**

Metabolism of morphine at 2 mM in HLM in the presence of Compound A at 0, 0.5, 1.0, 5.0, 10, 25, 50, 100, and 250 μM was evaluated. Formation of two glucuronide metabolites of morphine, M3G and M6G, was monitored. In the incubations containing 2 mM morphine, $IC_{50}$ of Compound A could not be estimated under the concentration range tested (Table 1.1 and Table 1.2; Figure 1, respectively).

Table 1.1 M3G Formation Activity of Human Liver Microsomes in the Presence and Absence of Compound A

| Compound A (μM) | M3G (μM) | Activity [a] (pmole/mg/min) | Mean | SD | % Control |
|---|---|---|---|---|---|
| 0 | 5.83 | 389 | 371 | 11.3 | 100.0 |
| | 5.69 | 379 | | | |
| | 5.44 | 363 | | | |

(continued)

| Compound A (μM) | M3G (μM) | Activity [a] (pmole/mg/min) | Mean | SD | % Control |
|---|---|---|---|---|---|
| 0.5 | 5.54 | 369 | 362 | 7.0 | 97.7 |
| | 5.32 | 355 | | | |
| | 5.45 | 363 | | | |
| 1.0 | 5.49 | 366 | 357 | 13.1 | 96.2 |
| | 5.45 | 363 | | | |
| | 5.13 | 342 | | | |
| 5.0 | 5.67 | 378 | 393 | 16.6 | 106.0 |
| | 6.17 | 411 | | | |
| | 5.87 | 391 | | | |
| 10 | 6.38 | 425 | 400 | 31.4 | 107.9 |
| | 5.47 | 365 | | | |
| | 6.17 | 411 | | | |
| 25 | 6.10 | 407 | 402 | 19.6 | 108.3 |
| | 6.27 | 418 | | | |
| | 5.70 | 380 | | | |
| 50 | 5.69 | 379 | 392 | 20.0 | 105.7 |
| | 6.23 | 415 | | | |
| | 5.73 | 382 | | | |
| 100 | 5.00 | 333 | 344 | 17.3 | 92.7 |
| | 5.46 | 364 | | | |
| | 5.02 | 335 | | | |
| 250 | 5.18 | 345 | 349 | 5.1 | 94.2 |
| | 5.22 | 348 | | | |
| | 5.33 | 355 | | | |
| [a]Protein concentration: 0.5 mg/mL; Incubation time: 30 min; Morphine concentration: 2mM | | | | | |

Table 1.2 M6G Formation Activity of Human Liver Microsomes in the Presence and Absence of Compound A

| Compound A (μM) | M6G (μM) | Activity [a] (pmole/mg/min) | Mean | SD | % Control |
|---|---|---|---|---|---|
| 0 | 0.789 | 52.6 | 51 | 1.5 | 100.0 |
| | 0.758 | 50.5 | | | |
| | 0.746 | 49.7 | | | |
| 0.5 | 0.765 | 51.0 | 49 | 2.2 | 96.9 |
| | 0.704 | 46.9 | | | |
| | 0.752 | 50.1 | | | |
| 1.0 | 0.723 | 48.2 | 48 | 1.7 | 94.4 |
| | 0.745 | 49.7 | | | |
| | 0.695 | 46.3 | | | |

(continued)

| Compound A (μM) | M6G (μM) | Activity [a] (pmole/mg/min) | Mean | SD | % Control |
|---|---|---|---|---|---|
| 5.0 | 0.742 | 49.5 | 52 | 2.8 | 102.4 |
| | 0.827 | 55.1 | | | |
| | 0.778 | 51.9 | | | |
| 10 | 0.869 | 57.9 | 56 | 2.1 | 110.4 |
| | 0.808 | 53.9 | | | |
| | 0.853 | 56.9 | | | |
| 25 | 0.764 | 50.9 | 54 | 3.3 | 105.8 |
| | 0.861 | 57.4 | | | |
| | 0.800 | 53.3 | | | |
| 50 | 0.833 | 55.5 | 55 | 4.5 | 108.3 |
| | 0.893 | 59.5 | | | |
| | 0.758 | 50.5 | | | |
| 100 | 0.733 | 48.9 | 50 | 2.8 | 97.4 |
| | 0.790 | 52.7 | | | |
| | 0.709 | 47.3 | | | |
| 250 | 0.727 | 48.5 | 49 | 1.6 | 96.5 |
| | 0.719 | 47.9 | | | |
| | 0.765 | 51.0 | | | |
| [a]Protein concentration: 0.5 mg/mL; Incubation time: 30 min; Morphine concentration: 2mM | | | | | |

Under the same conditions, positive control incubations with ketoconazole, a known inhibitor, demonstrated a ≥98.5% inhibition to both M3G and M6G activities.

### 4.5 Sample Analysis

Statistical calculations in the report tables were calculated from unrounded concentration values taken directly from the raw data. The concentration values appearing in the report tables were rounded for display purposes.

#### 4.5.1 Linearity

Calibration standard regression was performed by Analyste 1.5. Linearity was expressed as 2 the correlation coefficient ($r^2$) of the calibration curve. The calibration curve had correlation 2 coefficient ($r^2$) values ≥ 0.9924 for morphine, M3G, and M6G.

The linearity of morphine, M3G, and M6G were acceptable within the concentration range of 0.0175 to 3.5 μM, 0.01 to 2.0 μM, and 0.01 to 2.0 μM respectively.

#### 4.5.2 Specificity/Selectivity

All chromatograms obtained from blank sample were evaluated for the presence of any interference peaks at regions of interest of M3G, M6G, and the internal standard. No interference was observed in the region of interest. There was also no obvious interference between the analytes and internal standard. The results have shown that this method is very specific and selective.

The inhibition of UDP-glucuronosyl tranferase mediated metabolism of morphine glucuronidation by Compound A was investigated in human liver microsomes, by monitoring the formation of two prominent metabolites (morphine-3-glucuronide and morphine-6-glucuronide). Compound A showed no inhibition of morphine metabolism under the conditions employed in this *in vitro* study.

**Example 2: A clinical trial to assess the clinical benefit of Compound A as adjunctive treatment for persistent cancer pain**

The Study Primary objective is to evaluate the efficacy of Compound A 200 mg daily compared to placebo as adjunctive treatment for persistent cancer pain, as measured by the change of pain severity from baseline using the Numeric Rating Scale.

The Study Secondary Objectives are:

- to evalute the efficacy of Compound A compared to placebo as adjunctive treatment for persistent cancer pain, measured by :
    - reduction of pain intensity
    - responder rate
    - breakthrough pain frequency
    - background therapy utilization
    - mood
    - patient satisfaction of pain relief
    - constipation
    - heathcare utilization
    - quality of life,
- to evaluate the tolerability and safety of Compound A as adjunctive treatment for persistent cancer pain,
- to evaluate the blood concentration and byproducts of Compound A,
- to assess plasma endocannabinoid concentration.

The inclusion criteria are patient with moderate or severe, persistent cancer pain who are receiving the World Health Organization (WHO) Step 2 or 3 cancer pain treatment and :

- Pain generator (source of pain) must be primarily due to underlying cancer or cancer treatment,
- Pain generator (source of pain) must be classified as either primarily nociceptive or primarily neuropathic,
- Pain severity must be moderate or severe as defined by the Numeric Rating Scale during the screening phase.

The exclusion criteria are:

- Pain is not stable at study Visit 1 (screening visit) and Visit 2 (randomization visit),
- Use of prohibited adjuvant pain treatment in the week prior to Visit 1 or plan to use these medications during the study,
- Current use of medication containing tetrahydrocannabinol (THC),
- Chemotherapy within 4 weeks of Visit 1 or chemotherapy planned during the study,
- Radiotherapy within 4 weeks of Visit 1 or radiotherapy planned during the study (hemostatic palliative radiotherapy is permitted),
- Cancer related surgery within 4 weeks of Visit 1 or cancer-related surgery planned during the study.

For a given participating patient, the total duration of this study is 6 weeks:

Screening Phase:

- No investigational medicinal product administered
- Patient continues to receive WHO Step 2 or 3 cancer pain treatment as Background Therapy

Randomized Treatment Phase:

- Patients randomized to 1 of 2 treatment groups in a 1:1 ratio:

    Group I: [Background Therapy + Compound A]
    Group II: [Background Theray + placebo]

- Compound A is administered 200 mg once daily in the morning with food
- Patient continues to receive WHO Step 2 or 3 cancer pain treatment as Background Therapy

Posttreatment Phase:

- No investigational medicinal product administered
- Patient continues to receive WHO Step 2 or 3 cancer pain treatment as Background Therapy

The primary endpoint is a change from baseline (baseline = average pain intensity between Visit 1 and Visit 2) to end of treatment (end of treatment = average pain intensity during Week 4) in cancer pain severity assessed by the Numeric Rating Scale.

The secondary endpoints are:

- Adverse events and other clinical and para-clinical parameters (eg, vital signs, physical examinations, clinical laboratories, electrocardiogram)
- Other safety assessments (suicidality, drug abuse liability, cognitive function)
- Brief Pain Inventory Short-Form (BPI-SF)
- Responder rate
- Breakthrough pain frequency
- Background therapy utilization
- Mood
- Nausea visual analog scale
- Constipation.

## Claims

1. 2-amino-2-oxoethyl trans-3-[5-(6-methoxy-naphtalen-1-yl)-1,3-dioxan-2-yl]propyl-carbamate for use in the treatment of persistent cancer pain.

2. 2-amino-2-oxoethyl trans-3-[5-(6-methoxy-naphtalen-1-yl)-1,3-dioxan-2-yl]propyl-carbamate for use according to claim 1 wherein pain generator is primarily due to underlying cancer.

3. 2-amino-2-oxoethyl trans-3-[5-(6-methoxy-naphtalen-1-yl)-1,3-dioxan-2-yl]propyl-carbamate for use according to claim 1 wherein pain generator is primarily due to cancer treatment.

4. 2-amino-2-oxoethyl trans-3-[5-(6-methoxy-naphtalen-1-yl)-1,3-dioxan-2-yl]propyl-carbamate for use according to any of claims 1 to 3 wherein pain generator is classified as primarly nociceptive.

5. 2-amino-2-oxoethyl trans-3-[5-(6-methoxy-naphtalen-1-yl)-1,3-dioxan-2-yl]propyl-carbamate for use according to any of claims 1 to 3 wherein pain generator is classified as primarly neuropathic.

6. 2-amino-2-oxoethyl trans-3-[5-(6-methoxy-naphtalen-1-yl)-1,3-dioxan-2-yl]propyl-carbamate for use according to any of claims 1 to 5 wherein the persistent cancer pain is bone cancer.

7. 2-amino-2-oxoethyl trans-3-[5-(6-methoxy-naphtalen-1-yl)-1,3-dioxan-2-yl]propyl-carbamate for use according to any of claims 1 to 6 wherein the persistent cancer pain is metastatic cancer.

8. 2-amino-2-oxoethyl trans-3-[5-(6-methoxy-naphtalen-1-yl)-1,3-dioxan-2-yl]propyl-carbamate for use according to any of claims 1 to 7 wherein the persistent cancer pain is metastatic bone cancer.

9. 2-amino-2-oxoethyl trans-3-[5-(6-methoxy-naphtalen-1-yl)-1,3-dioxan-2-yl]propyl-carbamate for use in a therapeutic treatment to reduce cancer pain intensity.

10. 2-amino-2-oxoethyl trans-3-[5-(6-methoxy-naphtalen-1-yl)-1,3-dioxan-2-yl]propyl-carbamate for use in a therapeutic treatment to reduce breakthrough cancer pain frequency.

11. 2-amino-2-oxoethyl trans-3-[5-(6-methoxy-naphtalen-1-yl)-1,3-dioxan-2-yl]propyl-carbamate for use according to any of claims 1 to 10 as adjunctive treatment.

12. 2-amino-2-oxoethyl trans-3-[5-(6-methoxy-naphtalen-1-yl)-1,3-dioxan-2-yl]propyl-carbamate for use according to

any of claims 1 to 11 in a therapeutic treatment to reduce utilization of background therapy.

**13.** 2-amino-2-oxoethyl trans-3-[5-(6-methoxy-naphtalen-1-yl)-1,3-dioxan-2-yl]propyl-carbamate for use according to claim 11 or 12 wherein the background therapy is morphine or morphine derivative.

**14.** 2-amino-2-oxoethyl trans-3-[5-(6-methoxy-naphtalen-1-yl)-1,3-dioxan-2-yl]propyl-carbamate for use according to any of claims 1 to 13 wherein 2-amino-2-oxoethyl trans-3-[5-(6-methoxy-naphtalen-1-yl)-1,3-dioxan-2-yl]propyl-carbamate is administered 200 mg once daily.

**15.** Pharmaceutical composition comprising 2-amino-2-oxoethyl trans-3-[5-(6-methoxy-naphtalen-1-yl)-1,3-dioxan-2-yl]propyl-carbamate as active ingredient and pharmaceutically acceptable excipients for use according to any of claims 1 to 14.

**16.** An article of manufacture comprising:

- a packaging material;
- a pharmaceutical composition comprising 2-amino-2-oxoethyl trans-3-[5-(6-methoxy-naphtalen-1-yl)-1,3-dioxan-2-yl]propyl-carbamate as active ingredient and pharmaceutically acceptable excipients; and
- a label or package insert contained within the packaging material indicating that patients receiving the treatment with the above mentioned pharmaceutical composition can be treated for persistent cancer pain.

Figure 1 - Formation of M3G and M6G (Percent of Control) in Pooled Human Liver Microsome as a Function of Compound A Concentration

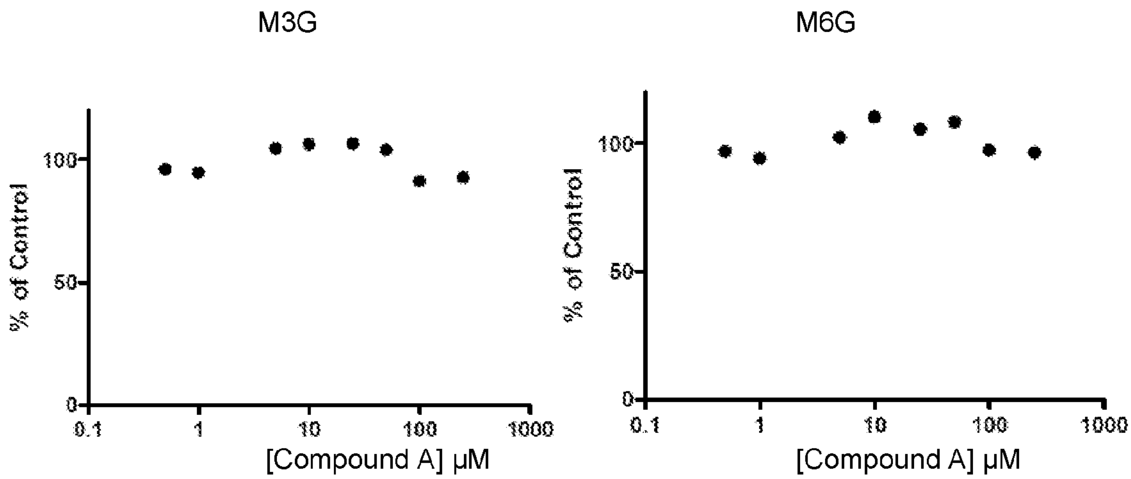


Protein concentration: 0.5 mg/mL; Incubation time: 30 min; Morphine concentration: 2 mM

Europäisches Patentamt
European Patent Office
Office européen des brevets

# EUROPEAN SEARCH REPORT

Application Number
EP 11 30 5903

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | I. A. KHASABOVA ET AL: "A Decrease in Anandamide Signaling Contributes to the Maintenance of Cutaneous Mechanical Hyperalgesia in a Model of Bone Cancer Pain", JOURNAL OF NEUROSCIENCE, vol. 28, no. 44, 29 October 2008 (2008-10-29), pages 11141-11152, XP055012168, ISSN: 0270-6474, DOI: 10.1523/JNEUROSCI.2847-08.2008 * abstract * * page 11141, left-hand column, paragraph 1 - page 11142, left-hand column, paragraph 2 * * page 11148, left-hand column, last paragraph - right-hand column, paragraph 1 * * page 11149, left-hand column, paragraph 3 * * page 11149, left-hand column, last paragraph - right-hand column, paragraph 2 * * page 11150, left-hand column, last paragraph * * figures 1c, 5d * * page 11145, right-hand column, paragraph 3 * ----- | 1-16 | INV. A61K31/357 A61K31/485 A61K45/06 A61P25/04 |
| X | WO 2004/020430 A2 (SANOFI SYNTHELABO [FR]; ABOUABDELLAH AHMED [FR]; BAS MICHELE [FR]; DAR) 11 March 2004 (2004-03-11) | 16 | |
| Y | * page 12; example 2; compound 49 * * page 22, line 1 - page 23, line 35 * * page 24, line 6 - line 22 * * claims 5,12,13 * ----- | 1-16 | |

TECHNICAL FIELDS SEARCHED (IPC)
A61K

The present search report has been drawn up for all claims

1

| Place of search | Date of completion of the search | Examiner |
|---|---|---|
| The Hague | 16 November 2011 | Gradassi, Giulia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

**ANNEX TO THE EUROPEAN SEARCH REPORT ON EUROPEAN PATENT APPLICATION NO.** EP 11 30 5903

This annex lists the patent family members relating to the patent documents cited in the above-mentioned European search report.
The members are as contained in the European Patent Office EDP file on
The European Patent Office is in no way liable for these particulars which are merely given for the purpose of information.

16-11-2011

| Patent document cited in search report | Publication date | Patent family member(s) | Publication date |
|---|---|---|---|
| WO 2004020430 A2 | 11-03-2004 | AR 041053 A1 | 27-04-2005 |
| | | AT 409184 T | 15-10-2008 |
| | | AT 434598 T | 15-07-2009 |
| | | AU 2003274292 A1 | 19-03-2004 |
| | | BR 0313846 A | 27-09-2005 |
| | | CA 2496040 A1 | 11-03-2004 |
| | | CA 2700319 A1 | 11-03-2004 |
| | | CN 1777595 A | 24-05-2006 |
| | | CN 101445504 A | 03-06-2009 |
| | | CN 101906071 A | 08-12-2010 |
| | | CO 5700820 A2 | 30-11-2006 |
| | | DK 1537096 T3 | 02-02-2009 |
| | | DK 1840125 T3 | 09-11-2009 |
| | | EP 1537096 A2 | 08-06-2005 |
| | | EP 1840125 A2 | 03-10-2007 |
| | | ES 2312808 T3 | 01-03-2009 |
| | | ES 2329181 T3 | 23-11-2009 |
| | | FR 2843964 A1 | 05-03-2004 |
| | | HK 1074445 A1 | 05-12-2008 |
| | | HK 1108157 A1 | 27-11-2009 |
| | | HR 20050190 A2 | 31-10-2005 |
| | | HR 20090270 A2 | 30-09-2009 |
| | | IL 166882 A | 30-12-2010 |
| | | IS 7699 A | 17-02-2005 |
| | | JP 4585854 B2 | 24-11-2010 |
| | | JP 2005539045 A | 22-12-2005 |
| | | JP 2010248218 A | 04-11-2010 |
| | | KR 20050059108 A | 17-06-2005 |
| | | KR 20100134807 A | 23-12-2010 |
| | | MA 27390 A1 | 01-06-2005 |
| | | MX PA05002319 A | 08-06-2005 |
| | | NZ 538404 A | 27-07-2007 |
| | | PL 209339 B1 | 31-08-2011 |
| | | PT 1537096 E | 11-12-2008 |
| | | PT 1840125 E | 29-09-2009 |
| | | RS P20050182 A | 21-09-2007 |
| | | RS P20100169 A | 30-04-2011 |
| | | SI 1537096 T1 | 28-02-2009 |
| | | SI 1840125 T1 | 31-12-2009 |
| | | UA 82847 C2 | 26-05-2008 |
| | | US 2005182130 A1 | 18-08-2005 |
| | | US 2006247290 A1 | 02-11-2006 |
| | | US 2009286868 A1 | 19-11-2009 |
| | | US 2010280076 A1 | 04-11-2010 |
| | | WO 2004020430 A2 | 11-03-2004 |
| | | ZA 200501537 A | 25-10-2006 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 11 30 5903

This annex lists the patent family members relating to the patent documents cited in the above-mentioned European search report.
The members are as contained in the European Patent Office EDP file on
The European Patent Office is in no way liable for these particulars which are merely given for the purpose of information.

16-11-2011

| Patent document cited in search report | Publication date | Patent family member(s) | Publication date |
|---|---|---|---|
| ------------------------------------------------------------------ | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 20040204303 A